(19) Europäisches Patentamt / European Patent Office / Office européen des brevets

(11) **EP 3 226 229 A1**

(12) **EUROPEAN PATENT APPLICATION**

(43) Date of publication:
**04.10.2017 Bulletin 2017/40**

(51) Int Cl.:
*G09B 19/00* (2006.01)   *A63B 24/00* (2006.01)

(21) Application number: **17164050.1**

(22) Date of filing: **31.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.03.2016  IT UA20162163**

(71) Applicant: **MEDIACLINICS S.r.l.**
**20851 Lissone (MB) (IT)**

(72) Inventor: **FUMAGALLI, Mario**
**20126 Milano (IT)**

(74) Representative: **Ferrari, Barbara**
**Botti & Ferrari S.r.l.**
**Via Cappellini, 11**
**20124 Milano (IT)**

(54) **MOTION EVALUATION METHOD AND SYSTEM IN A SPORT CONTEXT**

(57)    This document hereby describes a motion evaluation method in a sport context, said method including the steps of:
- providing at least one sensor (2) apt to detect parameters relating to an athlete (1); and
- recording a specific technical movement (MA) of said athlete (1) by means of a video recording device (4) in order to generate a video (V2), said specific technical movement (MA) starting at an instant (to);

The method further comprises the following steps:
- storing in a central unit (3) a pre-recorded video (V1) comprising a target specific technical movement (MT), said specific technical movement (MA) corresponding to said target specific technical movement (MT);
- sending said video (V2) to said central unit (3);
- univocally identifying said instant (to) via said at least one sensor (2), said instant (to) being communicated to said central unit (3);
- generating in said central unit (3) a comparison video (VS) wherein said pre-recorded video (V1) is associated with said video (V2), said pre-recorded video (V1) and said video (V2) being automatically synchronized so that said target specific technical movement (MT) and said specific technical movement (MA) start at a same instant in said comparison video (VS); and
- displaying said comparison video (VS) by means of a display device (5).

FIG. 4

## Description

Application Field

The herein discussed invention regards a motion evaluation method in a sport context. The invention notably regards a method that includes the steps of providing at least one sensor apt to detect parameters relating to an athlete, and a step of recording a specific technical movement of said athlete by means of a video recording device in order to generate a video, said specific technical movement starting in an specific instant.

The invention also regards a correlated motion evaluation system in a sport context; the following description is compiled with regards to this application field with the sole purpose of easing its presentation.

Invention Background

As is well known, it is important, in order to undertake an efficient process of improving one's technique in a sport, to have an exact and objective perception of one's own body and movements. This necessity is perceived all the more in those sports that require executing a specific technical movement following an exact sequence.

It has been found that there is a general absence of methods and systems that can facilitate and accelerate an athlete's performance improvement, regarding his or her sports technique in a specific sport.

The existence of and access, at a moderate price, to simple methods and systems intended to increase training efficiency could truly represent an efficient aid for many athletes, by, for example, speeding up the learning process of a specific technical movement, particular to a specific sport; also by simplifying the learning "ex novd' of a sport, and hereby facilitating the access to said sport for many new users.

For example, it is well known that athletes employ a personal trainer or coach that follows his or her training in all its stages.

However, the efficiency of a coach in the improvement of an athlete's technical performance is limited by the athlete's perception of the coach's analysis as a subjective, and thereby debatable, opinion.

A well-known technical solution to this problem, which aims to improve the efficacy of a training session, envisages a video recording of the athlete's performance, and specifically of a specific technical movement, so that the athlete's movements may be observed objectively.

While useful under various aspects, this solution presents several inconveniences, as it doesn't provide an immediate understanding of the technical errors performed by the athlete in the execution of said specific technical movement. Consequently, a solution such as this offers an athlete limited support for his technical improvement.

Furthermore, problems and limitations often arise due to the video recording not real-time availability and to its difficult manipulation, which mark this solution as not practical. By using this already known system, the athlete is not able to immediately correct his movements in light of the errors highlighted in the video recording and doesn't acquire the immediate perception of a technical improvement, which is actually a truly important aspect, especially from a psychological point of view.

Another well known solution in the training efficiency improvement field envisages the athlete using wearable movement sensors which provide a precise numerical analysis of the performed movement. However, the ease of use and the cost of these sensors are to date very far from making this solution relevant in a large-scale context.

It must also be stressed that all solutions known up to now do not envisage a comparison with an "ideal" technical movement, the so-called "by-the-book movement".

The U.S.A. patent application N. US 2011/0275045 A1 describes a system and method for the comparison of a video recording of a professional athlete in a specific sport, in which recording the professional athlete is depicted by a stick figure, and video recording of a beginner in the same sport. In said file, the videos are synchronized so that a specific, to-be-analized technical movement starts in the same instant, said two video recording being also possibly overlapped.

The technical problem of the invention in issue is to create a motion evaluation method in a sport context, whose features allow to overcome the limitations and inconveniences that currently afflict all already-known solutions, and which notably enables an athlete to learn and/or perfect a specific technical movement, said method being easily applicable, available at a moderate price and allow an objective, automatic and real-time comparison of the athlete's specific technical movement and the by-the-book specific technical movement, and thereby also allowing a real improvement of the training efficacy.

Invention Summary

The idea this invention is based on is to use synchronized sensors and recording devices to generate a comparison video, where a video including a specific technical movement performed by an athlete is automatically synchronized with a pre-recorded video including a target specific technical movement that is performed by a top player and can be considered a by-the-book movement, said specific technical movements starting at exactly the same instant in the comparison video.

On the basis of said idea, the above mentioned technical problem is solved by means of a motion evaluation method in a sport context, said method including the steps of:

- providing at least one sensor apt to detect parameters relating to an athlete;

- recording an athlete's specific technical movement by means of a video recording device in order to generate a video, said specific technical movement starting at a specific instant, also called primary instant;

- storing in a central unit a pre-recorded video including a target specific technical movement, said specific technical movement corresponding to the target specific technical movement;

- sending the athlete's video recording to the central unit;

- univocally identifying the instant in which the athlete's target specific technical movement starts by means of at least one sensor, said instant being communicated to the central unit;

- generating in the central unit a comparison video wherein the pre-recorded video is associated with the athlete's video, said pre-recorded video and said video being automatically synchronized in such a way that the target specific technical movement and the specific technical movement start at a same instant in the comparison video; and

- displaying the comparison video by means of a display device.

More specifically, the invention includes the following additional features, which can occur singularly or in combination with one another.

According to one aspect of the invention, the method can also include a step of synchronizing a target key frame of the pre-recorded video with a key frame of the athlete's video, said target key frame corresponding to the starting instant of the target specific technical movement and said athlete's key frame corresponding to the instant in which the athlete's specific technical movement starts.

The method according to the invention in issue can further include the steps of:

- generating a univocal time code in the central unit;

- sending the univocal time code from the central unit to the at least one sensor and to the video recording device; and

- synchronizing, through said univocal time code, said at least one sensor with the video recording device so as to univocally identify the instant in which the athlete's specific technical movement starts.

According to another aspect of the invention, the step of displaying said comparison video can occur in real time during the execution of said specific technical movement and/or be delayed compared to the execution of said specific technical movement by the athlete.

Furthermore, the step of generating said comparison video can comprise a step of a juxtaposing the pre-recorded video with the athlete's video.

The method of the invention in issue can further comprise the step of detecting physical parameters relating to a specific technical movement by means of at least one detection device.

It must also be added that the step of sending the univocal time code from the central unit 3 can comprise a step of sending said univocal time code to the at least one detection device.

Furthermore, the invention method can comprise the following steps:

- communicating from said central unit to said at least one detection device the instant in which the athlete's specific technical movement starts; and

- detecting, by means of said at least one detection device, physical parameters at an instant $t' = t_0 + t_\Delta$, which is delayed compared to the instant in which the athlete's specific technical movement starts, where $t_\Delta$ is a preset time interval, and/or detecting the maximum value of said physical parameters in said pre-set time interval.

Said method can further comprise a step of processing in said central unit the athlete's video within a time interval defined by an initial instant $t_i$ and by a final instant $t_f$ such that $t_i = t_0 - t_1$ and $t_f = t_0 + t_2$, which is to say that $t_1$ and $t_2$ can be advanced or delayed compared to the instant in which the athlete's specific technical movement starts, $t_1$ and $t_2$ being preset time constants.

According to still another aspect of the invention, the method can further comprise a step of communicating data from the at least one sensor, from the central unit, from the video recording device and the at least one detection device to the cloud system, and vice versa, so as to provide the athlete with specific application services.

Furthermore, it is hereby stressed that the target specific technical movement is performed by a professional or by a top player of a particular sport.

Lastly, the method of the invention further comprises a step of driving the playback of the comparison video by means of said at least one sensor, by performing specific gestures intended to drive the playback of said comparison video.

The invention furthermore relates to a motion evaluation system in a sport context, said motion evaluation system including:

- at least one sensor apt to detect parameters relating to an athlete; and

- a video recording device apt to record a specific technical movement of the athlete, generating a video, said specific technical movement starting at an specific instant;

said motion evaluation system being characterized in that it comprises:

a central unit connected to the at least one sensor and to the video recording device, said central unit comprising a storage unit that includes a pre-recorded video comprising a corresponding target specific technical movement, the athlete's video being sent to said central unit by the reception/transmission means of said central unit and of said video recording device, respectively, said at least one sensor comprising means apt to univocally identify the instant in which the athlete's specific technical movement starts, and reception/transmission means apt to communicate said instant to said central unit, said central unit further comprising a synchronization unit apt to synchronize said pre-recorded video with said video in order to generate a comparison video wherein said target specific technical movement and said specific technical movement are associated and start at a same instant; and
a display device apt to display said comparison video.

According to one aspect of the invention, the central unit can comprise means apt to generate a univocal time code, the reception/transmission means sending said univocal time code to said at least one sensor and to said video recording device for the synchronization of said at least one sensor and said video recording device.

According to another aspect of the invention, the system can further comprise at least one detection device apt to detect physical parameters relating to said specific technical movement and comprising reception/transmission means, the univocal time code and the instant in which the athlete's specific technical movement starts being communicated from the central unit to said at least one detection device through said reception/transmission means.

It is furthermore hereby stressed that said at least one detection device can be a mechanical sensor.

According to another aspect of the invention, the system can further comprise a cloud system connected to the at least one sensor, to the central unit, to the video recording device and to the at least one detection device via specific reception/transmission means.

Furthermore, said at least one sensor can be selected between an accelerometric sensor and a biometric sensor and can comprise a signal processor that is embedded therein and is apt to identify the instant in which the athlete's specific technical movement starts.

According to another aspect of the invention, the video recording device can be a high frame rate videocamera with a frame rate between 100fps and 120 fps.

It must also be added that the pre-recorded video and the athlete's video can be placed side by side in the comparison video.

Furthermore, the reception/transmission means of the at least one device and the central unit can be wireless.

Lastly, the at least one sensor is apt to drive the playback of the comparison video by means of the signal processor, which is programmed so as to automatically recognize specific gestures performed by the athlete, said gestures being intended to drive the playback of said comparison video.

The features and advantages of the system and method as described in the invention will result evident from the following description of an example of its implementation, which is given for information only and will not limit its future realization to the attached figures.

Brief Figures Description

In said figures:

- figure 1 schematically depicts a number of components of a motion evaluation system apt to realize the method of the motion evaluation system according to the invention in issue;

- figure 2 schematically depicts a different implementation of the motion evaluation system according to the invention in issue;

- figure 3 schematically depicts an example of a sequence of images from a comparison video generated in one of the steps of the motion evaluation method according to the invention in issue; and

- figure 4 schematically depicts an exemplary display device in the motion evaluation system according to the invention in issue.

Detailed Description

In reference to said figures, a motion evaluation method is below described destined to be used by consumers, and especially athletes, for the learning and/or improvement of specific technical movements in a sport context.

It is hereby stressed that said figures merely depict a schematic view and are not in scale, but are instead drawn so as to emphasize the most important features of the invention. Furthermore, in the figures the different components are represented schematically, their shape being subject to variations according to the desired application. Lastly, it is hereby stressed that in the figures identical reference numbers pertain to elements identical in shape or purpose.

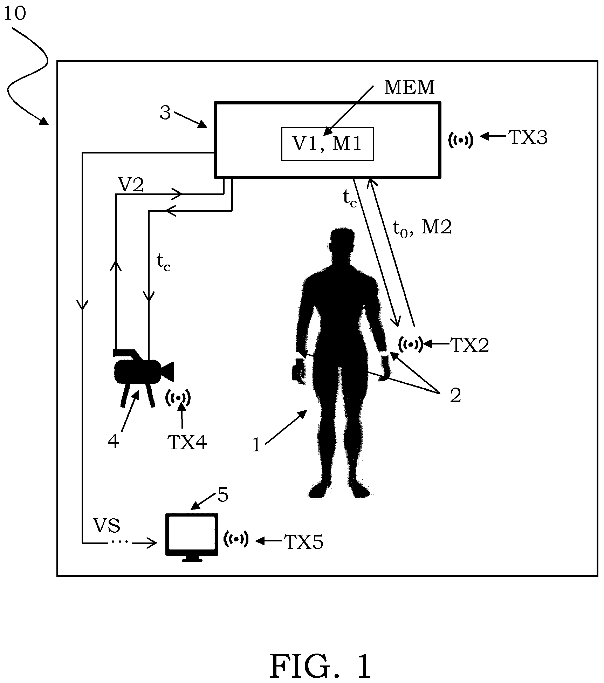

In its most general implementation, the motion evaluation method according to the invention in issue is based on processing and synchronizing data originated by a number of components of a motion evaluation system, as will be described below, with particular reference to figure 1.

More specifically, the motion evaluation method according to the invention in issue initially comprises at least one step of positioning at least one sensor 2 apt to detect parameters relating to an athlete 1.

Said at least one sensor 2 can be a motion detection sensor which detects the athlete 1's movement. For example, said at least one sensor 2 can be an accelerometric sensor positioned as a bracelet on the athlete 1's wrist, as depicted in figure 1; however the invention isn't limited in this respect, as said at least one sensor 2 can also be of any kind and positioned in any way that detects the athlete 1's movement. Furthermore, as depicted in figure 1, the method according to the invention in issue comprises the use of a number of sensors 2, and specifically two sensors (one of each wrist of athlete 1), but evidently the number and positioning of said sensors 2 can vary according to necessity and/or the sport in question, said figure only being provided as an example and not restricting in any way the capacity of the invention in issue.

The method of the invention also includes a step of recording a specific technical movement MA of the athlete 1 by means of a video recording device 4, thus generating a video V2, said specific technical movement MA starting at an instant to.

Beneficially according the invention in issue, the motion evaluation method further comprises the steps of:

- storing in a central unit 3 a pre-recorded video V1 comprising a corresponding target specific technical movement MT, the specific technical movement MA corresponding to said target specific technical movement MT;

- sending the video V2 to the central unit 3;

- univocally identifying the instant to by means of the sensors 2, said instant to being communicated to the central unit 3;

- generating in the central unit 3 a comparison video VS wherein the pre-recorded video V1 is associated with the video V2, said pre-recorded video V1 and said video V2 being synchronized so that the target specific technical movement MT and the specific technical movement MA start at a same instant in said comparison video VS; and

- displaying the comparison video VS by means of a display device 5.

The target specific technical movement MT, incorporated in the pre-recorded video V1 that has been stored in the central unit 3, corresponds to an "ideal" movement, realized by a top player 1' or expert athlete of a specific sport and properly recorded. Alternatively, the target specific movement MT can be performed by a expert trainer in said sport, properly recorded and then supplied, for example, to the athletes trained by said trainer, who is then considered the top player 1'.

So as to identify the starting instant of the target specific technical movement MT, the top player 1' has previously worn sensors such as sensors 2 and has performed, while being recorded by a video recording device, such as for example the video recording device 4, said target specific technical movement MT. Alternatively, said target specific technical movement MT can be included in a clip from a match in which said top player 1' has competed, the instant in which said specific technical movement has began being identified manually by a video analyst.

Notably, the instant in which said target specific technical movement MT starts is univocally established, identifying a target key frame K1 of said pre-recorded video V1, said target key frame K1 corresponding to the starting instant of said target specific technical movement MT. Said pre-recorded video V1 is then stored in said central unit 3 and is then available to every athlete 1 that might want to compare his own specific technical movement MA with said target specific technical movement MT and thus with a by-the-book movement, with the purpose of improving his sport technique.

It is hereby stressed that, in the description in issue, the phrasing "instant in which said specific target MT starts" indicates the actual start of the movement, or alternatively an instant univocally identifiable, such as for example the instant in which the athlete impacts the ball, the instant in which the ball is released, the instant in which the athlete's foot lifts off the ground, etc.

The step of recording the athlete 1's specific technical movement MA, generating the video V2, is realized by means of the video recording device 4, which is preferably a high frame rate video camera (for example, a webcam), with a frame rate between 100fps and 120 fps and which is positioned so as to record the athlete's specific technical movement as clearly as possible. The recording device high frame rate will guarantee at a later time a clear and fluid displaying of the athlete 1's movement, even in slow motion.

Notably, the video recording device 4 can record the athlete 1's entire movement during a training session, and it is thus evident that in said video recording V2 the athlete 1's specific technical movement, to be compared with the target specific technical movement MT of the top player 1' in the sport in issue, is also included. The athlete 1's position with respect to the video recording device 4 during the recording of the specific technical movement MA preferably corresponds to the top player 1"s (or the trainer's) position during the recording of the specific technical movement MT.

Notably, when the athlete 1 performs the specific technical movement MA, the sensors 2 univocally and exactly identify the instant to in which said specific technical movement starts, said instant to being instantly communicated by said sensors 2 to said central unit 3 via apt means of reception/transmission TX2, part of said sensors 2, that are preferably wireless, as is explained in detail below. It is hereby stressed that said central unit 3 also includes apt means of reception/transmission TX3 to receive/transmit data to/from said sensors 2 and said video recording device 4.

At the same time, the video recording device 4 transmits to the central unit 3 the video V2 by ulterior means of reception/transmission TX4, that are preferably wireless.

Specifically, the central unit 3 receives and processes said video V2 only within a time interval defined by an initial instant $t_i$ and a final instant $t_f$ defined by the following expressions:

$$t_i = t_0 - t_1;$$

and

$$t_f = t_0 + t_2,$$

wherein $t_1$ and $t_2$ are time constants with a previously-set tolerance, set so as to include in the video V2 the entire specific technical movement MA and not include other irrelevant movements. The constants $t_1$ and $t_2$ can clearly vary according to necessity and/or circumstances, whilst the instant to depends on the athlete 1's execution of said specific technical movement.

The step of univocally identifying the instant to that corresponds with the start of the specific technical movement MA is specifically realized by means of a signal processor that is associated with each and every sensor 2, said signal processor being for example embedded, and thus incorporated in each and every sensor 2, programmed with the necessary algorithms so as to analyze the athlete 1's movement and to automatically identify, recognize and isolate specific instants of said movement, such as said instant to, said signal processor not being illustrated in the figures as it is embedded in said sensors 2.

A step of generating a univocal time code $t_c$ in the central unit 3 is started, contemporary to the steps of recording said specific technical movement MA and of identifying said instant to, said univocal time code $t_c$ being sent by the reception/transmission means TX3 from said central unit 3 to the sensors 2 and to the video recording device 4, thus enabling the automatic synchronization of all data from said sensors 2 and said video recording device 4. Specifically, the univocal timecode $t_c$ enables the synchronization of the clocks of the sensors 2 and said video recording device 4, so as to create a common temporal base and thus enable the precise and univocal identification of said instant to via the signal processors associated with the sensors 2, and also the following exact synchronization of the target specific technical movement MT and the specific technical movement MA, as will be explained in detail below. Consequently, through the univocal timecode $t_c$ generated by the central unit 3, the instant $t_0$ is correctly associated with said video recording V2, the sensors 2 and video recording device 4 sharing the same temporal base.

Thus, the data transmitted by the sensors 2 and the video recording device 4 is equipped with the univocal time code $t_c$ and is processed in said central unit 3 to univocally identify, through the instant to, a key frame K2 of said video V2 corresponding to said instant to, and as such corresponding to the start of said specific technical movement and corresponding to the target key frame K1.

At the conclusion of the step in which the instant $t_0$ is identified, as soon as said instant to has been communicated to said central unit 3 the step of generating in said central unit 3 a comparison video VS immediately starts, in which video said pre-recorded video V1 is associated and synchronized with said video V2 in said central unit 3. Notably, said central unit 3 processes said video V2 received from the video recording device 4 and identifies the key frame K2, as previously described. Once the key frame K2 relating to the instant to has been identified, the central unit 3 synchronizes the pre-recorded video V1 and the video V2 so that the pre-recorded video V1 target key frame K1 is synchronized with the video recording V2 key frame K2, said key frame K2 relating to said instant to. This thus guarantees that the key frame K2 of the video V2 is later displayed in the same instant in which the target key frame K1 of said video recording V1 is displayed. Thus, in the comparison video VS, the top player 1"s target specific technical movement MT and the athlete 1's specific technical movement MA start at exactly the same instant and are displayed in the same moment.

The central unit 3 thus generates the comparison video VS, which includes both the pre-recorded video V1 and the video V2, said videos being synchronized and associated with one another, the target specific technical movement MT and the specific technical movement MA starting at exactly the same instant in said comparison video VS.

The method of the invention in issue thus ends with the step of displaying the comparison video VS by means of a display device 5, which can be connected to the central unit 3. The display device 5 can be for example a screen located in the place where the athlete 1 trains, i.e. a field projector, or it can be a device such as a personal computer, a tablet, a smartphone, a tv screen and many others devices.

By means of the comparison video VS the athlete's specific technical movement can thus be properly compared, instant by instant, with the target specific technical movement MT, the pre-recorded video V1 being preferably placed side by side with the video V2 in the step of generating said comparison video VS.

Beneficially according to the invention in issue, the step of displaying the comparison video VS can take place both in real time, i.e. during the execution of said specific technical movement MA, and at a later time, i.e. after the execution of said specific technical movement MA.

Notably, in one form of implementation of the invention in issue, the step of displaying the comparison video VS on the display device 5 takes place in real time, during the execution of said specific technical movement MA, so that the athlete 1 may compare his technique with the top player 1"s in real time and may thus immediately correct potential errors or imperfections in his specific technical movement MA. In this form of implementation, the display device 5 is preferably placed in front of the athlete 1 that performs the specific technical movement MA, but different arrangements are obviously also possible.

Furthermore, in this form of implementation, the display device 5 is connected to the central unit 3 by apt means of reception/transmission TX5, such as for example wireless means or a cable system, said means being such as to not bring any delay in displaying the comparison video VS.

Notably, in this form of implementation, the pre-recorded video V1, previously stored in the central unit 3, is appropriately maintained in stan-by mode exactly at the target key frame K1 and, in the moment in which the instant $t_0$ is communicated to said central unit 3, said pre-recorded video V1 and the video recording V2 can be displayed at the same time without any delay due to the processing of said pre-recorded video V1, the displaying of said comparison video VS thus starting from the target key frame K1 for said video V1 and from the key frame K2 for said video recording V2, so that the target specific technical movement MT and the specific technical movement MA start at exactly the same instant, namely the instant $t_0$ that triggers the generating and displaying of said video VS.

In some sports, it might be difficult for the athlete 1 to perform the specific technical movement MA and at the same time view the comparison video VS. For this reason, in an alternative form of implementation of the invention in issue, the step of displaying the comparison video VS is delayed compared to the execution of said specific technical movement MA, preferably by a few seconds. Notably, in this form of implementation, once the step of generating the comparison video VS is terminated, the method includes a step of storing said comparison video VS in the central unit 3 and the step of displaying said comparison video VS can thus be delayed compared to the execution of said specific technical movement MA and thus compared to the recording of said video recording V2, so as to allow the athlete 1 to view and evaluate his performance in a later moment.

To be specific, said central unit 3 includes a memory unit MEM apt to store the pre-recorded video V1 and the comparison video VS.

As previously stated, the comparison video VS is preferably displayed a few seconds after the execution of said specific technical movement MA, even though it can obviously be displayed at any time after its execution.

It as also obviously possible to execute the step of displaying the comparison video VS both in real time and in a later moment, after appropriate storing of said video.

Furthermore, the sensors 2, in addition to univocally identifying said instant to, can be used by the athlete 1 in the step of displaying the comparison video VS to control the displaying of said comparison video VS, the signal processor being aptly set so as to commute said sensors 2 from a "data acquisition" mode to a "control" mode. Notably, the signal processor embedded in the sensors 2 is programmed with specific algorithms that enable the automatic recognition of some of the athlete's specific gestures, said gestures being intended to drive the playback of said comparison video VS. For example, if the sensors 2 are bracelets positioned on the athlete 1's wrist, by rotating the wrist it is possible to perform basic commands on the comparison video VS such as play, pause, stop, forward and back, and also to determine the comparison video VS playback speed on the basis of, for example, the width of the wrist rotation.

In the step of generating the comparison video VS, the top player 1"s target specific technical movement MT is placed side by side and synchronized with the athlete 1's specific technical movement MA, said comparison video being displayable both in real time and at a later time, after appropriate storing of said video. The comparison video VS is beneficially easily manipulable, its storing in the MEM memory unit granting the possibly to browse through it and to regulate its playback speed so as to observe in detail the athlete 1's movement, with an ulterior possibility to highlight the specific technical movements MT and MA with specific markers, as will be explained in detail below.

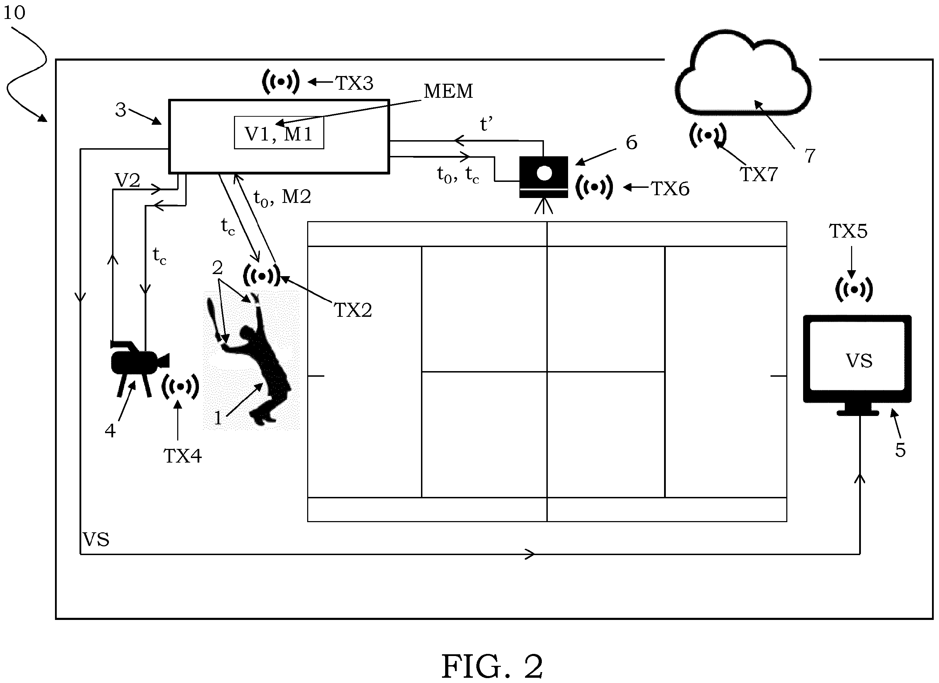

With reference now to figure 2, a variation of implementation of the invention motion evaluation method is below described, said variation being described with particular reference to tennis, the athlete 1 being thusly a tennis player.

It is however hereby stressed that the area of interest of the invention isn't limited to tennis, the method of the invention being applicable in numerous sports, such as golf, baseball, volleyball, fencing, canoeing and many other sports that require performing specific technical movements, said figure only being provided as an example and not restricting in any way the capacity of the invention in issue.

To be specific, according to said implementation variation, the invention method further comprises a step of detecting physical parameters relating to the specific technical movement MA by means of at least one detection device 6. Notably, said at least one detection device can be a mechanical sensor.

For example, said sport being tennis and said specific technical movement MA being the serve (or stroke), the at least one detection device 6 detects physical parameters of a ball after it has been hit by the athlete 1 with a racket. Notably, the at least one detection device 6 can be a device detecting the ball vector speed, a device detecting the ball rotation, an accelerometer, a videocamera or a high frame rate camera, a Doppler radar, only to mention a few.

As previously highlighted, the invention in issue is not restricted to the field of tennis but is applicable in many other sports, the transition from one sport to another possibly requiring the use of a different number of sensors 2 and/or their different positioning, as well as a different number of detection devices 6 and/or their different positioning. Furthermore, in specific implementations, the sensors 2 can be biometric sensors worn by the athlete 1, possibly combined with accelerometric sensors apt to detect said athlete 1's parameters such as posture, limb speed, heart rate and many other relevant parameters.

According to another implementation variation, not depicted in the figures, sensors embedded in the athlete 1's equipment are also envisaged, such as for example a racket and a ball in the case of tennis, or a ball, a bat, a mitt or a sword in the case of other sports such as football, golf, baseball and fencing, to name a few.

The univocal time code $t_c$ generated by the central unit 3 is obviously transmitted by said central unit 3 also to the detection device 6, which is in turn equipped with reception/transmission means TX6, so as to synchronize said detection device 6 with the sensors 2 and with the video recording device 4. It is thus possible for example, still with reference to tennis, to univocally and clearly detect important parameters such as the ball speed after a serve, and compare said speed with the top player 1"s ball speed after said top player has performed the target specific technical movement MT.

Notably, the central unit 3 transmits to the detection device 6 the instant to and the detection device 6 subsequently detects a physical parameter, such as the ball speed, in an instant $t' = t_0 + t_\Delta$, where $t_\Delta$ is a preset time interval previously defined by means of specific algorithms with which said detection device 6 is appropriately programmed. Said time t' is then transmitted from the detection device 6 via reception/transmission means TX6 to the central unit 3 to allow the comparison with the same physical parameter previously detected in connection with the n.1 top player. Alternatively, the physical parameter detected by the detection device 6 is the maximum value of said physical parameter in the previously defined time interval $t_\Delta$, said maximum value being determined via specific algorithms by the detection device 6 and being then sent to the central unit 3.

The central unit 3, the sensors 2, the video recording device 4 and possibly the detection device 6 can communicate via wireless means, but obviously other communication systems are possible. The communication between the sensors 2 and the central unit 3 preferably happens by wireless means, so as to avoid using cables.

Furthermore, it is also possible to include in the invention method a step of transmitting data from the sensors 2, the central unit 3, the video recording device 4 and the detection device 6, to a (web and/or mobile) cloud system and vice versa, so as to provide the athlete 1 with specific application services through additional transmission/reception means TX7. Access to the cloud server 7 occurs through dedicated client-server protocols and applications, specifically apt to transmit media files to a data storing server unit.

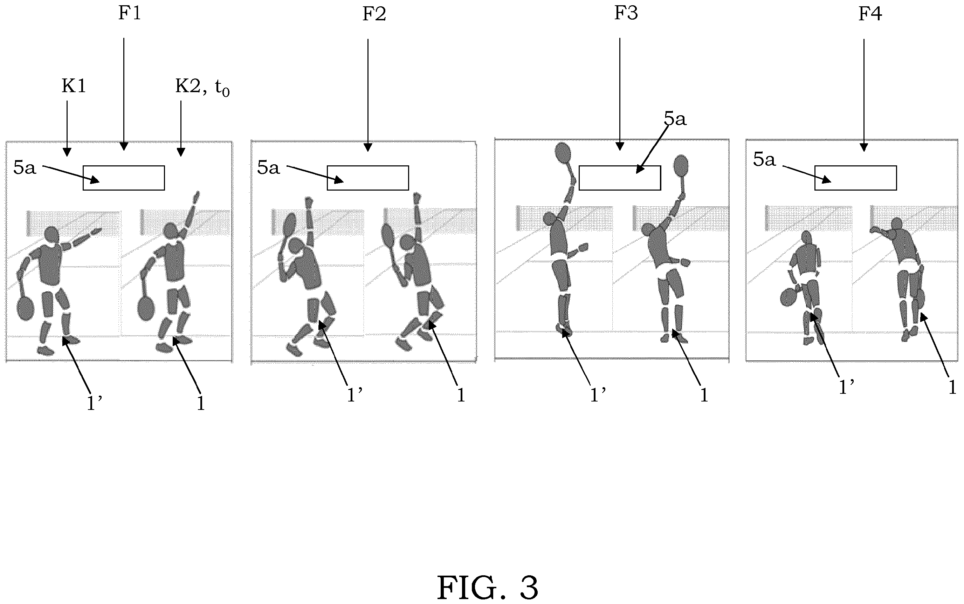

The method according to the invention will be below described, as depicted in figure 3, with regard to a specific tennis-related, non-restricting implementation example, and specifically with regard to the serve technique. Said figure schematically depicts a sequence of four-images (F1, F2, F3, F4) taken from the comparison video VS, from the releasing of the ball (Image F1) to the serve complete execution (image F4), in which the athlete 1's specific technical movement MA is placed side by side with the top player 1"s target specific technical movement MT.

To be specific, the athlete 1 is filmed with the video recording device 4 while he serves and the sensors 2, worn by the athlete on his wrists, detect the instant to in which the ball is released from the athlete 1's hand. As soon as the instant is communicated to the central unit 3, in said central unit 3 the pre-recorded video V1 and the video V2 are synchronized so that the athlete 1 may watch in real time on the display device 5, preferably placed in front of the athlete 1, the comparison video VS in which the target key frame K1 and the key frame K2 start in the same instant. The detection device 6 furthermore supplies the ball speed after the serve, said speed being possibly displayed on the display device 5 in a specific area 5a for data visualization.

As depicted in figure 3, it is therefore possible to compare instant by instant the serve performed by the top player 1' with the one performed by the athlete 1, from the moment in which the ball is released to the complete execution of said movement.

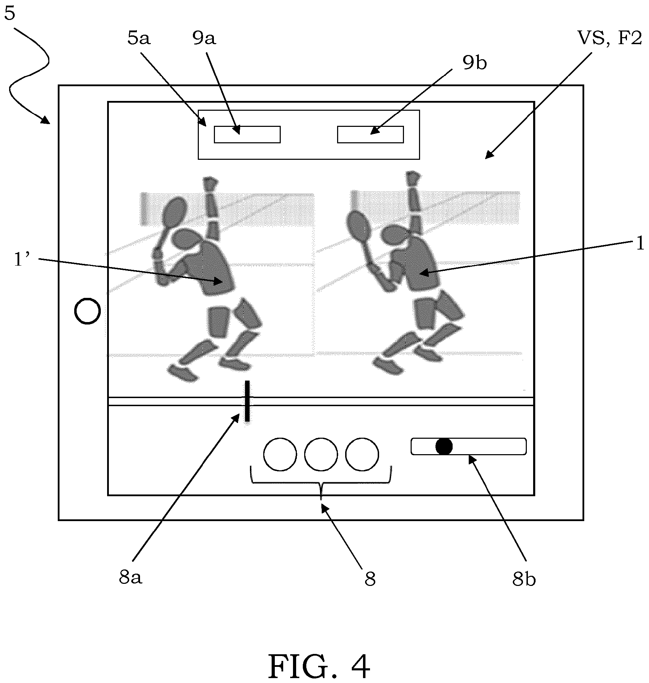

Lastly, figure 4 depicts an example of a display device 5, in which the image F2 from the comparison video VS is displayed. The display device 5 can be a touch-screen device and the display screen can comprise a multiple commands 8, said multiple commands 8 including the usual commands play, pause, stop, forward and back, and also a slider 8a to easily scroll through the frames of the comparison video VS; it can also include a comparison video VS playback rate control bar 8b, which allows a more thorough viewing of said comparison video's VS details, and notably a thorough analysis of the specific technical movements MA and MT, which may be highlighted by specific markers.

Two windows 9a and 9b are furthermore envisaged, displaying the top player 1"s and the athlete 1's physical parameters respectively, detected by the detection device 6, and comprising the area 5a for data visualization displayed by the display device 5.

The invention in issue also relates to a motion evaluation method in a sport context. Referring again to figure 1, such motion evaluation system is globally and systematically identified with the number 10.

The motion evaluation system 10 notably comprises at least one sensor 2 apt to detect parameters relating to an athlete 1.

As previously described, the at least one sensor 2 can be a motion detecting sensor such as, for example, an accelerometric sensor positioned as a bracelet on the athlete 1's wrist, as depicted in figure 1, or a biometric sensor apt to detect said athlete 1's parameters such as posture, limb speed, heart rate and many other relevant parameters. It is also hereby stressed that the motion evaluation system 10 can also envisage a combination of accelerometric and biometric sensors. It is also hereby stressed that the number and positioning of said sensors 2 can vary according to necessity and/or the sport in question.

Furthermore, the motion evaluation system 10 according to the invention in issue includes a video recording device 4 which records a specific technical movement MA of the athlete 1, generating a video V2, said specific technical movement MA starting in an instant to.

The video recording device 4 is preferably a high rate frame video camera, such as a webcam, with a frame rate preferably between 100 fps and 120 fps, and is positioned so as to record the athlete 1's specific technical movement as clearly as possible.

Beneficially according to the invention in issue, the motion evaluation system 10 further comprises a central unit 3 which comprises a storage unit MEM that includes a pre-recorded video V1, said pre-recorded video V1 including a target specific technical movement MT. It is hereby highlighted that the athlete 1's target specific technical movement MA corresponds to the target specific technical movement performed by a top player 1' in a specific sport, who for example has employed the sensors 2 and has been recorded with the video recording device 4, the pre-recorded video V1 being previously stored in the central unit 3 memory unit MEM.

The video recording device 4 is specifically placed so as to record the athlete 1, while he performs the specific technical movement MA, in a way that coincides as much as possible with the top player 1"s target specific technical movement recorded in the pre-recorded video V1 with regards to perspective, distance and height of the video recording device 4 compared to the athlete 1. The video V2 is transmitted from the video recording device 4 by transmission/reception means TX4 to the central unit 3, in which in turn are included transmission/reception/transmission means TX3.

The sensors 2 include means apt to univocally identify the instant to, which coincides with the start of the specific technical movement MA, and to communicate said instant to to the central unit 3 via transmission/reception means TX2. To be specific, the sensors 2 include a signal processor, which can for example be therein embedded and thus incorporated in the sensors 2, and is programmed with the necessary algorithms so as to analyze the athlete 1's movement and to automatically identify, recognize and isolate specific instants of said movement, such as the instant to.

As previously mentioned, the central unit 3, the sensors 2 and the video recording device 4 are equipped respectively with transmission/reception means TX3, TX2 and TX4 to communicate and transmit data to each other. Said transmission/reception means can for example be wireless, but other communication systems are also possible. The communication between the sensors 2 and the central unit 3 preferably happens via wireless means, so as to avoid the use of cables.

The central unit 3 includes a synchronization unit which synchronizes the pre-recorded video V1 with the video V2 in order to generate a comparison video VS in which said pre-recorded video V1 and said video V2 are associated and the target specific technical movement MT and the specific technical movement MA start at a same instant. The pre-recorded video V1 and the video V2 are preferably placed side by side in the comparison video VS. Said comparison video VS can be stored in the central unit 3 memory unit MEM in which the pre-recorded video V1 was previously stored, so as to also allow its displaying at a later time.

The motion evaluation system 10 lastly includes a display device 5 to displays the comparison video VS, said display device 5 being possibly connected to the central unit 3 by apt transmission/reception means TX5. The display device 5 can be for example a screen located in the place where the athlete 1 trains, i.e. a field projector, or it can be a device such as a personal computer, a tablet, a smartphone, a tv screen and many other devices.

Furthermore, the display device 5 can be a touch-screen device and the display screen can comprise multiple commands 8, said multiple commands 8 comprising the usual commands play, pause, stop, forward and back, and also a slider 8a to easily scroll through the frames of the comparison video VS; it can also include a comparison video VS playback rate control bar 8b, which allows a more thorough viewing of said comparison video's VS details, and notably a thorough analysis of the specific technical movement MA and MT, which may be highlighted by specific markers. An area 5a for data visualization is furthermore envisaged, comprising two windows 9a and 9b displaying respectively the top player 1"s and the athlete 1's physical parameters detected by the detection device 6.

It is obviously foreseeable that said display device 5 may include commands 8, a slider 8a and/or a physical parameters playback rate control bar 8b.

The central unit 3 includes means apt to generate a univocal timecode $t_c$ which, at the launch of the motion evaluation system 10, is transmitted by transmission/reception means TX3 from said central unit 3 to the sensors 2 and the video recording device 4, said univocal timecode $t_c$ enabling the automatic synchronization of the sensors 2 clocks and the video recording device 4 clock, so that the instant to can be univocally identified by means of the sensors 2 and can be correctly associated with the video 2.

In the implementation variation depicted in figure 2, the motion evaluation system 10 further comprises at least one detection device 6 apt to detect apt to detect physical parameters relating to the specific technical movement MA. Notably, said at least one detection device 6 can be a mechanical sensor. As previously stated, it is hereby highlighted that although figure 2 depicts a tennis player and the detection device 6 is apt to detect parameters such as the tennis ball speed and/or rotation, said figure is not in any way restricting the capacity of the invention in issue, as the motion evaluation system 10 can be applied to several sports, varying for each sport the number and/or the placement of the sensors 2 and the detection device 6.

The univocal timecode $t_c$ generated by the central unit 3 is evidently also transmitted from said central unit 3 to the detection device 6, so as to synchronize said detection device 6 with the sensors 2 and the video recording device 4, said detection device 6 comprising transmission/reception means TX6, preferably wireless. It is hereby stressed that the instant $t_0$ is also communicated from the central unit 3 to the detection device 6 via the above mentioned transmission/reception means. Alternatively, the detection device 6 constantly transmits the collected data to the central unit 3 which only takes into consideration meaningful data, which is to say the data included in the time interval between $t_0$ and $t' = t_0 + t_\Delta$.

Furthermore, the motion evaluation system 10 can include a web and/or mobile cloud system 7, which provides the athlete 1 with specific application services, said cloud system 7 being connected to the sensors 2, the central unit 3, the video recording device 4 and the detection device 6 by apt transmission/reception means TX7. Access to the cloud server 7 occurs through dedicated client-server protocols and applications, specifically apt to transmit media files to a data storing server unit.

It is thus evident that the motion evaluation system 10 according to the invention in issue firstly allows the synchronization of the sensors 2, the video recording device 4 and possibly the detection device 6 through the univocal timecode $t_c$ generated by the central unit 3, said motion evaluation system 10 lastly granting the synchronization and objective comparison between the data acquired by the above-mentioned components and a pre-recorded ideal model.

In conclusion, the method and system according to the invention in issue allow to overcome the problems of systems already in existence by acquiring objective data relating to an athlete and providing said athlete with an objective comparison of his or her specific technical movement and the target specific technical movement performed by a top player of a sport of interest, said movements being synchronized automatically and in real time with one another via a central unit and displayed by a display device.

Beneficially according to the invention in issue, the submitted method and system come at a particularly moderate price, as they exploit a combination of components based on pre-existing and well-established, and also easily usable, technologies, the acquisition and synchronization of all data from the various components being effectuated automatically.

Through the use of sensors, such as for example movement sensors and/or biometric sensors that can be worn by the athlete, and through the use of detection devices, such as for example mechanical sensors, synchronized with one another by means of a univocal timecode generated by the central unit, it is possible to obtain objective and exact data with the purpose of identifying the athlete's specific technical movement, detecting significant parameters relating to a specific sport and, lastly, comparing said movement and said parameters with those of a top player of said sport, who is considered to perform a so-called "by-the-book" manual.

The invention in issue thus supplies a new training and evaluation tool to athletes of various sports, providing them with the possibility of analyzing their training session and evaluating it in detail via the method and system described above.

Accordingly, the generation of the univocal timecode in the central unit permits to identify, recognize and isolate specific segments in the athlete's cinematic movement and to thus univocally and correctly identify the synchronization key frames.

As previously stressed, the invention in issue allows both a real time display of the comparison video and a display delayed compared to the execution of the specific technical movement, said comparison video being stored in the central unit. In the latest case, the possibility is envisaged to drive the comparison video playback display, for example through the sensors attached to the athlete's wrists. For example, it is possible to regulate the comparison video playback rate on the display device so as to allow a detailed observation of the specific technical movement, which may be highlighted by specific markers.

The use of a high frame rate video recording device also permits a clear and fluid video visualization, even in slow motion.

Compared to the solutions known to date, the invention in issue allows athletes to train more efficiently by detecting significant parameters through the acquisition and processing of objective data, said significant parameters being compared with those of a top player in a specific sport in real time and/or at a later time. Through the comparison of the specific technical movements recorded by the video recording device and the data detected by the mechanical sensors, the athlete can thus easily evaluate his or her performance and his or her progress objectively, either independently or with the support of a personal trainer.

This brings the advantage of reducing the time span required to perfect and/or acquire a specific technical movement of a specific sport.

Lastly, it is hereby stressed that the method and system here introduced are substantially international, as they do not present any difficulty of employment connected with the language or the social and cultural aspects of different countries.

**Claims**

1.  A motion evaluation method in a sport context, said method including the steps of:

    - providing at least one sensor (2) apt to detect parameters relating to an athlete (1); and
    - recording a specific technical movement (MA) of said athlete (1) by means of a video recording device (4) in order to generate a video (V2), said specific technical movement (MA) starting at an instant (to);

    said method being **characterized in that** it further comprises the steps of:

    - storing in a central unit (3) a pre-recorded video (V1) comprising a target specific technical movement (MT), said specific technical movement (MA) corresponding to said target specific technical movement (MT);
    - sending said video (V2) to said central unit (3);
    - univocally identifying said instant (to) by means of said at least one sensor (2), said instant (to) being communicated to said central unit (3);
    - generating in said central unit (3) a comparison video (VS) wherein said pre-recorded video (V1) is associated with said video (V2), said pre-recorded video (V1) and said video (V2) being automatically synchronized in such a way that said target specific technical movement (MT) and said specific technical movement (MA) start at a same instant in said comparison video (VS); and
    - displaying said comparison video (VS) by means of a display device (5).

2.  The method according to claim 1, wherein said step of generating said comparison video (VS) comprises a step of synchronizing a target key frame (K1) of said pre-recorded video (V1) with a key frame (K2) of said video (V2), said target key frame (K1) relating to a starting instant of said target specific technical movement (MT) and said key frame (K2) relating to said instant (to).

3.  The method according to claim 1 or 2, further comprising the steps of:

    - generating a univocal time code ($t_c$) in said central unit (3);
    - sending said univocal time code ($t_c$) from said central unit (3) to said at least one sensor (2) and to said video recording device (4); and
    - synchronizing, through said univocal time code ($t_c$), said at least one sensor (2) with said video recording device (4) so as to univocally identify said instant (to).

4.  The method according to claim 1, wherein:

    said step of generating said comparison video (VS) comprises a juxtaposition of said pre-recorded video (V1) with said video (V2); and/or said step of displaying said comparison video (VS) occurs in real time during the execution of said specific technical movement (MA) and/or is delayed compared to the execution of said specific technical movement (MA).

5.  The method according to claim 3, further comprising the steps of:

    - detecting physical parameters relating to said specific technical movement (MA) by means of at least one detection device (6), wherein said step of sending said univocal time code ($t_c$) from said central unit (3) comprises a step of sending said univocal time code ($t_c$) to said at least one detection device (6);
    - communicating said instant (to) from said central unit (3) to said at least one detection device (6); and
    - detecting, by means of said at least one detection device (6), physical parameters at an instant $t' = t_0 + t_\Delta$, where $t_\Delta$ is a preset time interval, and/or detecting a maximum value of said physical parameters in said preset time interval.

6.  The method according to any one of the preceding claims, further comprising a step of processing in said central unit (3) said video (V2) within a time interval defined by an initial instant $t_i$ and by a final instant $t_f$ such that $t_i = t_0 - t_1$ and $t_f = t_0 + t_2$, $t_1$ and $t_2$ being preset time constants.

7.  The method according to any one of the preceding claims, wherein said target specific technical movement (MT) is performed by a professional or by a top player (1') of a particular sport.

8. The method according to any one of the preceding claims, further comprising a step of driving the playback of said comparison video (VS) by means of said at least one sensor (2), by performing specific gestures intended to drive the playback of said comparison video (VS).

9. A motion evaluation system (10) in a sport context, said motion evaluation system (10) comprising:

at least one sensor (2) apt to detect parameters relating to an athlete (1); and
a video recording device (4) apt to record a specific technical movement (MA) of said athlete (1), said video recording device (4) generating a video (V2), said specific technical movement (MA) starting at an instant (to);
said motion evaluation system (10) being **characterized in that** it comprises:

a central unit (3) connected to said at least one sensor (2) and to said video recording device (4), said central unit (3) comprising a storage unit (MEM) that includes a prerecorded video (V1) comprising a corresponding target specific technical movement (MT), said video (V2) being sent to said central unit (3) by means of reception/transmission means (TX3, TX4) of said central unit (3) and of said video recording device (4), respectively, said at least one sensor (2) comprising means apt to univocally identify said instant (to) and reception/transmission means (TX2) apt to communicate said instant (to) to said central unit (3), said central unit (3) further comprising a synchronization unit apt to synchronize said pre-recorded video (V1) with said video (V2) in order to generate a comparison video (VS) wherein said target specific technical movement (MT) and said specific technical movement (MA) are associated and start at a same instant; and
a display device (5) apt to display said comparison video (VS).

10. The motion evaluation system (10) according to claim 9, **characterized in that** said central unit (3) comprises means apt to generate a univocal time code ($t_c$), said reception/transmission means (TX3) sending said univocal time code ($t_c$) to said at least one sensor (2) and to said video recording device (4) for the synchronization of said at least one sensor (2) and said video recording device (4).

11. The motion evaluation system (10) according to claims 9 or 10, **characterized in that** it further comprises at least one detection device (6) apt to detect physical parameters relating to said specific technical movement (MA) and comprising reception/transmission means (TX6), said univocal time code ($t_c$) and said instant (to) being communicated from said central unit (3) to said at least one detection device (6) through said reception/transmission means (TX6).

12. The motion evaluation system (10) according to any one of the claims from 9 to 11, **characterized in that** it further comprises a cloud system (7) connected to said at least one sensor (2), to said central unit (3), to said video recording device (4) and to said at least one detection device (6) through specific reception/transmission means (TX7).

13. The motion evaluation system (10) according to any one of the claims from 9 to 12, **characterized in that**:

said at least one sensor (2) is selected from an accelerometric sensor and a biometric sensor; and/or
said at least one detection device (6) is a mechanical sensor.

14. The motion evaluation system (10) according to any one of the claims from 9 to 13, **characterized in that** said at least one sensor (2) comprises a signal processor that is embedded therein and is apt to identify said instant (to).

15. The motion evaluation system (10) according to claim 14, **characterized in that** said at least one sensor (2) is apt to drive the playback of said comparison video (VS) by means of said signal processor, which is programmed so as to automatically recognize specific gestures performed by said athlete (1), said gestures being intended to the driving of the playback of said comparison video (VS).

FIG. 1

FIG. 2

EP 3 226 229 A1

FIG. 3

FIG. 4

Europäisches
Patentamt

European
Patent Office

Office européen
des brevets

## EUROPEAN SEARCH REPORT

Application Number

EP 17 16 4050

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | David Bayles: "Sprongo Demonstration - Comparisons and Overlays", , 11 September 2012 (2012-09-11), XP055309787, Retrieved from the Internet: URL:https://www.youtube.com/watch?v=JBN10LxvQ0o [retrieved on 2016-10-12] * the whole document * | 1-15 | INV. G09B19/00 A63B24/00 |
| X | WO 2010/085704 A1 (BHUPATHI SHIV KUMAR [US]; FOERSTER WILLIAM MICHAEL [US]) 29 July 2010 (2010-07-29) * figures 2-6 * | 1-15 | |
| X,D | US 2011/275045 A1 (BHUPATHI SHIV KUMAR [US] ET AL) 10 November 2011 (2011-11-10) * paragraph [0007]; figures 2,3,6 * | 1-15 | |

TECHNICAL FIELDS
SEARCHED      (IPC)

G09B
A63B

The present search report has been drawn up for all claims

| Place of search | Date of completion of the search | Examiner |
|---|---|---|
| Munich | 7 July 2017 | Beker, Harald |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 16 4050

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2017

| Patent document cited in search report | Publication date | Patent family member(s) | Publication date |
|---|---|---|---|
| WO 2010085704 A1 | 29-07-2010 | NONE | |
| US 2011275045 A1 | 10-11-2011 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110275045 A1